# EUROPÄISCHE PATENTSCHRIFT

(11) **EP 2 001 433 B1**
(45) Veröffentlichungstag und Bekanntmachung des Hinweises auf die Patenterteilung: **03.01.2018**
(21) Anmeldenummer: 07702431.3
(22) Anmeldetag: 13.02.2007
(51) Int. Cl.: A61K 8/34, A61K 8/36, A61K 8/37, A61K 8/55, A61K 8/63, A61K 8/68, A61K 8/02, A61Q 19/00

(54) **TOPISCH ZU APPLIZIERENDE ZUSAMMENSETZUNG**
COMPOSITION FOR TOPICAL APPLICATION
COMPOSITION POUR APPLICATION TOPIQUE

(30) Priorität: 31.03.2006 DE 102006015544
(43) Veröffentlichungstag der Anmeldung: 17.12.2008
(73) Patentinhaber: Kuhs GmbH, 79541 Lörrach (DE)
(72) Erfinder: ALBRECHT, Martin, 51519 Odenthal (DE)
(74) Vertreter: Vossius & Partner Patentanwälte Rechtsanwälte mbB
(86) Internationale Anmeldenummer: PCT/DE2007/000259
(87) Internationale Veröffentlichungsnummer: WO 2007/112712

(56) Entgegenhaltungen:
- EP-A- 1 236 462
- WO-A-00/59454
- WO-A-2005/107691
- DE-A1- 19 713 793
- US-A- 4 999 348
- US-A- 6 022 561
- US-A1- 2003 012 762
- US-A1- 2005 238 677

## Beschreibung

Die vorliegende Erfindung betrifft eine topisch zu applizierende Zusammensetzung zur Anwendung beim Säugling oder Kleinkind mit den Merkmalen des Oberbegriffs des Patentanspruchs 1.

Topisch zu applizierende Zusammensetzungen, die bei Säuglingen oder Kleinkindern angewendet werden, lassen sich grundsätzlich unter Berücksichtigung ihrer Konsistenz einerseits in die lotionartigen Zusammensetzungen und andererseits in die cremeartigen Zusammensetzungen, die unterschiedliche Viskositäten aufweisen können, einteilen. Hierbei bewirken die lotionartigen Zusammensetzungen, daß der Haut des Kleinkindes bzw. des Säuglings relativ viel Wasser zugeführt wird, um so den insbesondere in den ersten Lebensjahren des Kindes auftretenden transdermalen Wasserverlust auszugleichen, während die cremeartigen Zusammensetzungen neben dem Ausgleich des transdermalen Wasserverlustes noch abhängig von ihrer jeweiligen chemischen Inhaltsstoffe eine okklusive Wirksamkeit besitzen, derart, daß sie die damit behandelten Hautbereiche gegenüber äußerlichen Beeinflussung, die auch als Noxe bezeichnet werden und die beispielsweise Urin betreffen, absperren.

Gemeinsam haben jedoch beide zuvor beschriebenen bekannten Zusammensetzungen den entscheidenden Nachteil, daß sie oftmals in einer relativ hohen Konzentration Tenside enthalten, wobei hier insbesondere die anionischen oder nichtionischen kurzkettigen Emulgatoren bevorzugt in den bekannten Zusammensetzungen enthalten sind. Diese Tenside bzw. Emulgatoren bewirken jedoch, daß die auf der Haut vorhandenen Oberflächenfette, die insbesondere die Hornschicht weich und geschmeidig halten und sie hydrophob machen, die überwiegend freien Fettsäuren, die den Säuremantel und somit eine weitere Schutzfunktion der Haut darstellen, und/oder die insbesondere im Stratum corneum vorhandenen Interzellular-Lipide, die kanalartig und verzweigt die oberen Hautschichten durchziehen und die die Zellen mörtelartig verbinden, emulgiert und/oder gelöst werden, mit der Folge, daß die so emulgierten bzw. gelösten Oberflächenfette, freien Fettsäuren sowie Interzellular-Lipid die Haut nicht mehr schützen können und somit auch keine ausreichende physikalisch-chemische Barriere der Haut zur Verfügung stellen. Dies wiederum führt dazu, daß eine derartig behandelte Haut, insbesondere bei Kindern, Kleinkindern und Säuglingen, bei der die zuvor geschilderte physikalisch-chemische Barriere erst im Auf- bzw. Ausbau begriffen ist, sehr empfänglich in bezug auf entzündliche Prozesse ist, hervorgerufen beispielsweise durch einen erhöhten transdermalen Wasserverlust oder durch äußerlich einwirkende Noxe, so daß insbesondere auch beim Säugling, Kleinkind oder Kind Hautirritationen, Rötungen, Juckreiz oder weitere ernste Erkrankungen, wie z.B. Ekzeme, Psoriasis oder Neurodermitis, keine Seltenheit mehr sind.

Aus der US 2005/0238677 ist eine Zusammensetzung bekannt, die als Trägersubstanz eine Mischung aus einem ersten Zuckerester, einem zweiten Zuckerester sowie einem festen Fettalkohol mit einem Schmelzpunkt von wenigstens 45 °C aufweist. Diese bekannte Trägersubstanzmischung, ist ein Öl-in-Wasser-Emulsionssystem, das ein biomimetisches multilamellares flüssigkristallines Gelnetzwerk ausbildet, wobei diese Veröffentlichung völlig offen läßt, welche Struktur sich hierhinter verbirgt.

Ebensowenig beschreiben die EP 1 236 462 A1 oder die US 4,999,348 die Struktur der dort benannten multilamellaren Emulsion oder definiert den hier verwendeten Begriff der lamellaren flüssigkristallinen Struktur bzw. flüssigkristallhaltigen Zusammensetzungen, während die DE 197 13 793 A1 auf eine lamellare Emulsion gerichtet ist, deren Emulsionströpfchen von einer flüssigkristallinen, lamellaren Phase aus Lipidmolekülen und Wasser umgeben sind, was nichts anderes besagt, daß es sich hierbei um Liposome handelt. Ebenso beschreibt die WO 2005/107691 A1 liposomale Systeme, während die EP 1 165 019 B1 auf lamellare flüssige Körperreinigungsmittel gerichtet ist, die entweder mizellare Strukturen oder geordnete flüssige kristalline, nicht planare Phasen aufweisen.

Eine Zusammensetzung mit den Merkmalen des Oberbegriffes des Patentanspruchs 1 ist aus der US 6,022,561 A bekannt, wobei die bekannte Zusammensetzung eine lamellare flüssigkristalline Struktur aufweist, die aus alternierenden Schichten aus Glactosylacylglycerinen und polarem Lösungsmittel besteht. In der einleitenden Beschreibung zu dieser Schrift sind lamellare Strukturen in Form von Vesikeln, die üblicherweise auch als Liposome bezeichnet werden, sowie Flüssigkristalle benannt, die als Trägersubstanz ein Phospholipid, das eine Doppelschicht ausbildet, aufweisen. Ausdrücklich stellt diese Schrift in Spalte 1, Zeilen 29 bis 36 heraus, daß Phosphatidylcholin aus Eigelb oder Sojabohnen für das optimale Quellen in Wasser und die Bildung von flexiblen Doppelschichten, die flüssigkristalline lamellare Strukturen aufbauen, ein wenig zu lipophil sei. Wie diese flüssigkristallinen lamellaren Strukturen, die bei Verwendung des Phosphatidylcholins entstehen, tatsächlich aussehen, läßt die Beschreibungseinleitung zu dieser Schrift offen.

Schließlich wird noch auf die gattungsfremde US 2003/0012762 A1 verwiesen, die eine spezielle Zusammensetzung zur Behandlung von geschädigter Haut, hervorgerufen durch Peeling oder Laserbehandlung, beschreibt. Diese Zusammensetzung dient zur Behandlung der Haut von älteren erwachsenen Menschen, die ihrer durch Peeling oder Laserbehandlung geschädigten Altershaut ein jugendliches Aussehen verleihen wollen. Die dort beschriebene Zusammensetzung liegt als lamellares Öl-in-Wasser-System vor.

Der vorliegenden Erfindung liegt die Aufgabe zugrunde, eine topisch zu applizierende Zusammensetzung, die zur Anwendung bei Säuglingen oder Kleinkindern geeignet ist, zur Verfügung zu stellen, mit der der Auf- bzw. Ausbau der physikalisch-chemischen Barriere der Haut besonders erfolgreich und wirksam unterstützt und gefördert wird.

Diese Aufgabe wird erfindungsgemäß durch eine topisch zu applizierende Zusammensetzung mit dem kennzeichnenden Merkmal des Patentanspruchs 1 gelöst.

Die erfindungsgemäße topisch zu applizierende bzw. anzuwendende Zusammensetzung, die zur Anwendung beim Säugling, Kleinkind oder beim Kind geeignet ist, weist eine hydrophile Flüssigkeit, mindestens einen anti-entzündlichen Wirkstoff sowie mindestens eine Trägersubstanz für den Wirkstoff auf, wobei die Trägersubstanz mit der hydrophilen Flüssigkeit lamellare, schichtartig ausgerichtete Doppelmembrane ausbildet oder zumindest in der Lage ist, diese insbesondere bei einer Verdünnung mit einer hydrophilen Flüssigkeit und vorzugsweise mit Wasser auszubilden. Bei diesen schichtartig übereinander angeordneten lamellaren Doppelmembrane erfolgt die Ausrichtung der einzelnen Trägersubstanzschichten derart, daß die hydrophilen Reste der Trägersubstanz jeweils nach außen und somit zu der diese Trägersubstanzschichten umgebenden hydrophilen Phase weisen, während die lipophilen Reste der Trägersubstanz zueinander nach innen ausgerichtet sind, was allgemein auch als Doppelmembrane bezeichnet wird. Orientieren sich hierbei abhängig von der Konzentration der Trägersubstanz in der erfindungsgemäßen Zusammensetzung dann mindestens zwei Doppelmembranschichten sandwichartig übereinander, so entstehen geschichtete Doppelmembranen, wobei diese geschichteten Doppelmembrane bevorzugt zwei bis zehn parallel zueinander ausgerichtete, überwiegend planar verlaufende und jeweils durch mindestens eine Schicht der hydrophilen Flüssigkeit getrennte Doppelmembranen enthalten. Sowohl die zuvor beschriebene einzelne Doppelmembrane als auch die geschichteten Doppelmembranen liegen als lamellare Strukturen in der erfindungsgemäßen Zusammensetzung vor und werden in der vorliegenden Beschreibung zusammengefaßt als lamellare, schichtartig ausgerichtete Doppelmembrane oder auch kurz als lamellare Doppelmembrane bezeichnet. Klarstellend ist anzumerken, daß in der erfindungsgemäßen Zusammensetzung neben den lamellaren, schichtartig ausgerichteten Doppelmembranen auch andere Strukturen, so beispielsweise vesikuläre und somit kugelförmige Einfachmembranstrukturen oder Mehrfachmembranstrukturen, vorliegen können, wobei jedoch diese vesikulären Strukturen in der erfindungsgemäßen Zusammensetzung allenfalls in untergeordnetem Maße auftreten und somit nicht die Struktur der erfindungsgemäßen Zusammensetzung charakterisieren.

Desweiteren ist zu der erfindungsgemäßen Zusammensetzung festzuhalten, daß bei der erfindungsgemäßen Zusammensetzung der anti-entzündliche Wirkstoff innerhalb der lamellaren Doppelmembrane und damit zwischen den nach innen gerichteten lipophilen Reste der Trägersubstanz homogen dispergiert ist, wobei diese homogene Dispersion vorzugsweise sowohl monomolekularen anti-entzündlichen Wirkstoff als auch oligomolekularen anti-entzündlichen Wirkstoff jeweils enthalten kann, derart, daß vorzugsweise nur ein Wirkstoffmolekül oder wenige Wirkstoffmoleküle diese Dispersion ausbilden. Mit anderen Worten ist der anti-entzündliche Wirkstoff bevorzugt mono-oder oligodisper innerhalb der Doppelmembrane und dort vorzugsweise im Bereich der lipophilen Reste der Trägersubstanz homogen verteilt, so daß die die Doppelmembrane umgebende hydrophile Flüssigkeit weitestgehend oder völlig frei von anti-entzündlichem Wirkstoff ist.

Als anti-entzündlicher Wirkstoff wird dieser aus der Gruppe ausgewählt, die Ursolsäure, Soja-Sterol, 18-beta-Glycyrrhetinsäure, Gamma-Oryzanol, Ferula-Säure umfaßt, wobei die erfindungsgemäße Zusammensetzung eine hohe pharmazeutische Wirksamkeit besitzt, die sie besonders geeignet macht, prophylaktisch und therapeutisch gegen eine Vielzahl von entzündlichen Hautprozessen eingesetzt zu werden, so insbesondere Hautrötungen, Juckreiz, Ekzeme, Psoriasis oder Neurodermitis sowie bei erhöhtem transdermalem Wasserverlust und äußerlich einwirkenden Noxen.

Ferner enthält die erfindungsgemäße Zusammensetzung als Trägersubstanz ein hydriertes Phospholipid mit einer Phasenübergangstemperatur über 30 °C und unter 70 °C. Hierbei ist die Phasenübergangstemperatur so definiert, daß sie die Temperatur bezeichnet, an der das kristalline System der Trägersubstanz in ein flüssiges System der Trägersubstanz übergeht, wobei vielfach diese Temperatur keine konkrete Einzeltemperatur darstellt sondern durch einen Temperaturbereich gekennzeichnet ist. So liegt beispielsweise die Phasenübergangstemperatur für das besonders bevorzugte hydrierte Phosphatidylcholin, das aus Sojabohnen isoliert ist und das eine Phosphatidylcholin-Konzentration von 93 + 3 Gew.% aufweist und dessen Acylreste zu 85 Gew.% aus Stearinsäure und zu 14 Gew.% aus Palmitinsäure bestehen, zwischen 54 °C und 58 °C und beträgt insbesondere 56 °C.

Die erfindungsgemäße Zusammensetzung weist eine Reihe von Vorteilen auf.

So ist zunächst festzuhalten, daß die erfindungsgemäße Zusammensetzung aufgrund ihrer lamellaren Struktur und der damit verbundenen Ähnlichkeit mit der Struktur der Oberflächenfette und insbesondere der Interzellular-Lipide besonders wirksam mit dazu beiträgt, daß die physikalisch-chemische Barriere bei Kindern, Kleinkindern und Säuglingen auf- bzw. ausgebaut wird, so daß hierdurch wirksam verhindert wird, daß einerseits ein transdermaler Wasserverlust im verstärktem Maße auftritt und andererseits daß äußerlich einwirkende Noxe in die Haut eindringen und dort die zuvor beschriebenen entzündlichen Prozesse hervorrufen. Bedingt dadurch, daß die erfindungsgemäße Zusammensetzung völlig auf Tenside oder sonstige Emulgatoren verzichtet, treten bei Anwendung der erfindungsgemäßen Zusammensetzung auch nicht die eingangs beim Stand der Technik beschriebnen Löse- und Emulgiereffekte der Oberflächenfette, der freien Fettsäuren und/oder der Interzellular-Lipide auf, so daß durch Anwendung der erfindungsgemäßen Zusammensetzung die physikalisch-chemische Barriere gestärkt und nicht, wie beim Stand der Technik, geschwächt wird. Bedingt dadurch, daß der in der erfindungsgemäßen Zusammensetzung innerhalb der lamellaren Doppelmembrane enthaltene anti-entzündliche Wirkstoff einerseits feinverteilt und andererseits in einer Struktur angeboten wird, der der lamellaren Struktur der natürlichen Lipidbarrieren der Haut nachempfunden ist, werden bereits beim Auftreten der ersten Ansätze eines entzündlichen Prozesses diese anti-entzündlichen Wirkstoffe wirksam, so daß hierdurch sehr effektiv derartige Erkrankungen abgewehrt werden, so daß der erfindungsgemäßen Zusammensetzung eine prophylaktische Wirkung zuzusprechen ist.

Selbst wenn entsprechende Erkrankungen bereits aufgetreten sind, so daß die erfindungsgemäße Zusammensetzung therapeutisch eingesetzt wird, sind diese entzündlichen Hautprozesse innerhalb kürzester Zeit nach Anwendung der erfindungsgemäßen Zusammensetzung einer Heilung zuzuführen, so daß die erfindungsgemäße Zusammensetzung nicht nur eine hohe prophylaktische sondern auch eine hohe therapeutische Wirksamkeit besitzt. Desweiteren konnte festgestellt werden, daß die Hautelastizität bei Anwendung der erfindungsgemäßen Zusammensetzung signifikant verbessert und/oder die Hautrauhigkeit bereits nach wenigen Tagen der Anwendung erheblich reduziert und die Hautfeuchtigkeit erhöht werden konnten, so daß die erfindungsgemäße Zusammensetzung nicht nur besonders wirksam in bezug auf die zuvor angesprochenen therapeutischen und prophylaktischen Eigenschaften bei den dort genannten Erkrankungen sondern desweiteren auch eine hohe kosmetische Wirksamkeit besitzt, die sich in einer Reduzierung der Hautrauhigkeit, Erhöhung der Hautelastizität, Erhöhung der Hautfeuchtigkeit ausdrückt und sich damit die erfindungsgemäße Zusammensetzung auch zur Pflege von trockener Haut insbesondere bei Kindern, Kleinkindern und Säuglingen besonders eignet.

Bezüglich des in der erfindungsgemäßen Zusammensetzung enthaltenen anti-entzündlichen Wirkstoffs ist festzuhalten, daß der anti-entzündliche Wirkstoff, der in der erfindungsgemäßen Zusammensetzung innerhalb der Doppelmembrane eingebettet und dort homogen dispergiert ist, ein solcher anti-entzündlicher Wirkstoff ist, der in der hydrophilen Flüssigkeit, die die Doppelmembrane umgibt, schlecht löslich ist, so daß diese hydrophile Flüssigkeit im wesentlichen keine oder nur in einer geringen Konzentration diesen anti-entzündlichen Wirkstoff aufweist. Hierdurch wird insbesondere verhindert, daß bei einer Anwendung beim Kleinkind oder Säugling eine zu hohe Konzentration an anti-entzündlichen Wirkstoffen mittels der hydrophilen Flüssigkeit auf die Haut des Kindes appliziert wird, wodurch eine zusätzliche Belastung der Haut des Kleinkindes bzw. des Säuglings vermieden wird.

Desweiteren ist zu diesem anti-entzündlichen Wirkstoff festzuhalten, daß vorteilhafterweise dieser anti-entzündliche Wirkstoff in der Trägersubstanz als solche ebenfalls schlecht löslich ist. Erst durch Zusammenwirkung der geeigneten hydrophilen Flüssigkeit mit der geeigneten Trägersubstanz wird dann eine solche Ausgestaltung erreicht, in der der anti-entzündliche Wirkstoff innerhalb der lamellaren Doppelmembrane dispergiert ist, wie dies zuvor ausgiebig für die erfindungsgemäße Zusammensetzung beschrieben ist. Eine derartige ideale und homogene Verteilung des anti-entzündlichen Wirkstoffes wird dabei darauf zurückgeführt, daß der in der hydrophilen Flüssigkeit schlecht lösliche Wirkstoff und in der eigentlichen Trägersubstanz ebenfalls schlecht lösliche Wirkstoff dann insbesondere in die mono- bzw. oligodisperse Form überführt wird, wenn die Trägersubstanz mit der hydrophilen Flüssigkeit zusammenwirkt und somit die Trägersubstanz als Dispergiermittel für den anti-entzündlichen Wirkstoff dient. Bedingt dadurch, daß der anti-entzündliche Wirkstoff in der Trägersubstanz als solche ebenfalls schlecht löslich ist, wird wirksam verhindert, daß ein derartiger anti-entzündlicher Wirkstoff dann zusammen mit der Trägersubstanz in tiefe Hautschichten penetriert bzw. permeiert und möglicherweise in die Blutgefäße gelangt, was ebenfalls sowohl bei Kleinkindern als auch insbesondere bei Säuglingen im hohen Maße unerwünscht ist.

Der Begriff schlecht löslich soll insbesondere so verstanden werden, daß der anti-entzündliche Wirkstoff bei 20 °C nur eine geringe Löslichkeit oder unlöslich in Wasser und der jeweiligen Trägersubstanz ist.

Bezüglich der Konzentration, in der der anti-entzündliche Wirkstoff in der erfindungsgemäßen Zusammensetzung enthalten ist, ist grundsätzlich festzuhalten, daß sich diese Konzentration danach richtet, für welche Hautstörungen bzw. Hauterkrankungen die erfindungsgemäße Zusammensetzung angewendet wird, sei es therapeutisch oder prophylaktisch, oder ob die erfindungsgemäße Zusammensetzung mehr im kosmetischen Bereich verwendet wird. Allgemein ist hierzu festzuhalten, daß geringe Konzentrationen an anti-entzündlichen Wirkstoffen immer dann in der erfindungsgemäßen Zusammensetzung enthalten sind, wenn diese eher im kosmetischen Bereich zur Pflege und Gesunderhaltung der Haut, insbesondere bei Säuglingen und Kleinkindern, angewendet wird, während für therapeutische Zwecke hier höhere Konzentrationen an anti-entzündlichem Wirkstoff in der erfindungsgemäßen Zusammensetzung enthalten sind. Üblicherweise variiert die Konzentration des anti-entzündlichen Wirkstoffs in der erfindungsgemäßen Zusammensetzung zwischen 0,01 Gew.% und 5 Gew.% und vorzugsweise zwischen 0,1 Gew.% und 2,5 Gew.%, bezogen auf die anwendungsfertige Zusammensetzung, wobei die zuvor wiedergegebenen grundsätzlichen Überlegungen dann abhängig von der jeweiligen Anwendung die Konzentration des anti-entzündlichen Wirkstoffes in der jeweiligen erfindungsgemäßen Zusammensetzung begrenzen.

Wie bereits vorstehend wiederholt dargelegt, weist die erfindungsgemäße Zusammensetzung eine hydrophile Flüssigkeit auf. Insbesondere ist in der erfindungsgemäßen Zusammensetzung als hydrophile Flüssigkeit Wasser enthalten, wobei der Begriff Wasser nicht nur destilliertes Wasser, entionisiertes Wasser oder osmotisch gereinigtes Wasser, sondern auch alle wäßrigen Systeme, so beispielsweise auch Puffersysteme oder Salzlösungen, abdeckt.

Besonders geeignet ist es jedoch, wenn in der erfindungsgemäßen Zusammensetzung als Trägersubstanz, die in der Lage ist, mit der hydrophilen Flüssigkeit und insbesondere mit Wasser die zuvor beschriebenen lamellaren Doppelmembranstrukturen auszubilden, mindestens ein hydriertes Phosphatidylcholin enthalten ist. Hier konnte nämlich festgestellt werden, daß das hydrierte Phosphatidylcholin einerseits im hohen Maße mit der hydrophilen Flüssigkeit bzw. mit dem Wasser lamellare Doppelmembranstrukturen ausbildet und andererseits diese lamellaren Doppelmembranstrukturen hervorragend geeignet sind, in die interzellularen Lipide der Haut und insbesondere in die interzellularen Lipide der Hornschicht zu wandern und dort den Aufbau bzw. Ausbau dieser interzellularen Lipidschicht zu unterstützen, während gleichzeitig eventuell auftretende entzündliche Prozesse besonders wirksam unterdrückt werden. Ferner hat das hydrierte Phosphatidylcholin den weiteren Vorteil, daß sie besonders stabile Zusammensetzungen ausbilden, die einerseits gegenüber chemischen und insbesondere oxidativen Angriff widerstandsfähig sind und andererseits eine hohe physikalische Stabilität und somit eine extrem große Lagerstabilität besitzen.

Bevorzugt wird jedoch ein solches hydriertes Phospholipid und insbesondere ein solches hydriertes Phosphatidylcholin in der erfindungsgemäßen Zusammensetzung vorgesehen, bei dem alle Acylreste ausschließlich oder überwiegend gesättigt sind, so daß insbesondere nur noch ungesättigte Acylreste in einer Konzentration kleiner als 10 Gew.% und vorzugsweise weniger als 5 Gew.% und ganz bevorzugt weniger als 1,5 Gew.% in dem hydrierten Phospholipid und/oder insbesondere in dem hydrierten Phosphatidylcholin vorhanden sind.

Klarstellend sei angemerkt, daß der Begriff Phospholipid selbstverständlich nicht nur ein einzelnes Phospholipid sondern auch ein Gemisch von Phospholipiden abdeckt, wobei das Phospholipid bzw. das Phospholipidgemisch natürlichen oder synthetischen Ursprungs sein kann. Ebenso selbstverständlich ist es, daß das Phospholipid nicht nur im vorstehenden Sinne hydriert sein kann sondern daß anstelle dieses hydrierten Phospholipids ein synthetisches Phospholipid eingesetzt wird, bei dem die Acylreste im vorstehenden Sinne alle oder überwiegend gesättigt sind.

Die vorstehend beschriebenen Vorteile besitzen solche Weiterbildungen der erfindungsgemäßen Zusammensetzung im verstärkten Maße, die als Trägersubstanz ein hydriertes Phospholipid enthalten, das mindestens 60 Gew.% und vorzugsweise zwischen 70 Gew.% und 95 Gew.% hydriertes Phosphatidylcholin aufweist, wobei sich diese Konzentrationsangaben auf die Konzentration des hydrierten Phospholipids beziehen, das als solches in der anwendungsfertigen erfindungsgemäßen Zusammensetzung als Trägersubstanz enthalten ist.

Bezüglich der Konzentration der in der erfindungsgemäßen Zusammensetzung enthaltenen Trägersubstanz, die mit der hydrophilen Flüssigkeit bzw. insbesondere auch mit dem Wasser die eingangs beschriebenen lamellaren Doppelmembranen ausbildet, ist allgemein festzuhalten, daß sich diese Konzentration danach richtet, für welchen Zweck die erfindungsgemäßen Zusammensetzung angewendet wird und welchen anti-entzündlichen Wirkstoff sie enthält. Desweiteren richtet sich die Konzentration der Trägersubstanz nach der chemischen Art der jeweils ausgewählten Trägersubstanz und ferner danach, welche Konzentration an lamellaren Doppelmembranen innerhalb der erfindungsgemäßen Zusammensetzung enthalten sein soll. Insbesondere ist die mindestens eine Trägersubstanz in der erfindungsgemäßen pharmazeutischen Zusammensetzung in einer Konzentration zwischen 0,5 Gew.% und 30 Gew.%, vorzugsweise in einer Konzentration zwischen 0,7 Gew.% und 5 Gew.%, bezogen auf die anwendungsfertige Zusammensetzung, vorhanden.

Eine weitere, bevorzugte Ausgestaltung der erfindungsgemäßen Zusammensetzung sieht vor, daß diese Ausgestaltung neben dem mindestens einen anti-entzündlichen Wirkstoff ferner noch einen weiteren Wirkstoff enthält, der aus der Gruppe ausgewählt ist, die Antiallergika, Antibiotika, Antimykotika, Dermatika, Antiseptika, durchblutungsfördernde Wirkstoffe, Vitamine und Wundbehandlungsmittel jeweils allein oder in Mischung umfaßt. Hierdurch wird es möglich, einerseits die Vorteile der erfindungsgemäßen Zusammensetzung, die auf den Auf- und Ausbau der physikalisch-chemischen Barriere gerichtet sind, mit einer anti-entzündlichen Wirkung zu paaren und dann diese Vorteile mit den Vorteilen zu kombinieren, die durch die zuvor aufgeführten weiteren Wirkstoffe herbeigeführt werden. Desweiteren liegt der Vorteil dieser Ausgestaltungen der erfindungsgemäßen Zusammensetzung darin, daß insbesondere auch bei der sehr empfindlichen Haut des Säuglings oder Kleinkindes allergische und/oder entzündliche Hautreaktionen selbst dann nicht auftreten, wenn der jeweilig weitere Wirkstoff dafür bekannt ist, daß er bei üblichen topischen Behandlungen bei einer Vielzahl von Anwendern Hautirritationen hervorrufen kann.

Insbesondere weist die erfindungsgemäße Zusammensetzung ein lokal wirkendes Dermatikum als weiteren Wirkstoff auf. Hierbei handelt es sich wahlweise um ein einzelnes Dermatikum als auch um eine Mischung von verschiedenen Dermatika, je nachdem, für welchen Anwendungszweck die erfindungsgemäße Zusammensetzung eingesetzt wird. Soll beispielsweise diese Ausführungsform der erfindungsgemäßen Zusammensetzung zur Behandlung von infizierten, gereizten oder erkrankten Hautbereichen, so zum Beispiel zur Behandlung von Ekzemen, Brandwunden, Dekubitus, Abszessen oder Pilzerkrankungen angewendet werden, so weist diese Ausführungsform der erfindungsgemäßen Zusammensetzung vorzugsweise als weiteren Wirkstoff einen solchen Wirkstoff auf, der aus der Gruppe, umfassend Tetracyclin, Erythromycin, Chlortetracyclin, Framycetinsulfat, Fusidinsäure, Fusidinsäuresalze, Chloramphenicol, Clindamycin, Gentamicinsulfat, Neomycin, Cortison und Cortisonderivate, insbesondere Hydrocortison und/oder Cortisol, Chlorokresol, Econazol, Miconazol, Thioconazol, Griseofulvin und Clotrimazol ausgewählt ist.

Bezüglich der Konzentration des weiteren Wirkstoffes in der erfindungsgemäßen Zusammensetzung ist festzuhalten, daß dieser weitere Wirkstoff insbesondere in einer Konzentration zwischen 0,05 Gew.% und 20 Gew.%, vorzugsweise in einer Konzentration zwischen 0,1 Gew.% und 4 Gew.%, bezogen auf das Gewicht der anwendungsfertigen Zusammensetzung, vorhanden ist.

Wie bereits zuvor beschrieben ist, sieht eine besondere bevorzugte Ausführungsform der erfindungsgemäßen Zusammensetzung vor, daß diese Wasser als hydrophile Flüssigkeit aufweist. Hierbei variiert, je nach Anwendung, die Konzentration des Wasser in der erfindungsgemäßen Zusammensetzung zwischen 5 Gew.% und 90 Gew.%, bezogen auf das Gewicht der anwendungsfertigen Zusammensetzung, wobei diese Konzentrationen auch bevorzugt für andere hydrophile Flüssigkeiten gelten.

Abhängig von dem jeweils vorgesehenen Anwendungsgebiet und der jeweils ausgesuchten Trägersubstanz sowie des jeweils anzuwendenden anti-entzündlichen Wirkstoffs sieht eine vorteilhafte Weiterbildung der erfindungsgemäßen Zusammensetzung vor, daß diese mindestens einen Alkohol, insbesondere einen mehrwertigen Alkohol, aufweist, wobei selbstverständlich hier als Alkohole solche Alkohole ausgewählt werden, die keine oder nur eine äußerst geringe Hautirritation hervorrufen, wobei diese Hautirritation dann, sofern sie überhaupt auftritt, durch den anti-entzündlichen Wirkstoff unmittelbar beseitigt bzw. kompensiert wird.

Als besonders geeignete Alkohole haben sich in der erfindungsgemäßen Zusammensetzung Pentylenglykol, Caprylyl Glykol, Glycerin oder Mischungen der zuvor genannten Alkohole erwiesen, so daß dementsprechend diese Alkohole und insbesondere die zuvor beschriebene Dreiermischung aus Pentylenglykol, Caprylyl Glykol und Glycerin in der erfindungsgemäßen Zusammensetzung enthalten sind.

Abhängig von dem jeweiligen Einsatzgebiet der erfindungsgemäßen Zusammensetzung kann diese neben dem anti-entzündlichen Wirkstoff und dem weiteren Wirkstoff noch mindestens ein N-Acyl-Alkanolamin und vorzugsweise N-Acyl-Ethanolamin enthalten, wobei dieses N-Acyl-Alkanolamin dafür bekannt ist, daß es ebenfalls anti-entzündliche Eigenschaften besitzt. Hierbei variiert die Konzentration des N-Acyl-Alkanolamins und insbesondere des N-Acyl-Ethanolamins zwischen 0,01 Gew.% und 10 Gew.%, vorzugsweise zwischen 0,1 % und 3 %, jeweils bezogen auf das Gewicht der anwendungsfertigen Zusammensetzung.

Insbesondere dann, wenn das N-Acyl-Alkanolamin einen C₁-C₁₈-Acylrest, vorzugsweise einen linear gesättigten und/oder ungesättigten C₁-C₁₈-Acylrest, aufweist, lassen sich mit einer derartigen Weiterbildung der erfindungsgemäßen Zusammensetzung besonders gut extrem auftretende entzündliche Hautreizungen oder Hauterkrankungen behandeln, zumal diese Weiterbildung der erfindungsgemäßen Zusammensetzung dann einerseits den in der Doppelmembrane eingelagerten anti-entzündlichen Wirkstoff, der auch als wirkstoffgemisch vorliegen kann, und andererseits das N-Acyl-Alkanolamin und insbesondere ein N-Acyl-Ethanolamin enthält, wobei letztere dafür bekannt sind, daß es insbesondere auch zur Behandlung von Hautreizungen, Hautirritationen, Hautrötungen und Hautbrennen eine gute pharmazeutische Wirksamkeit besitzt.

Insbesondere wird in den zuvor beschriebenen Ausführungsformen der erfindungsgemäßen Zusammensetzung, die das N-Acyl-A1-kanolamin enthalten, dieses N-Acyl-Alkanolamin aus der Gruppe ausgewählt, die N-Acetyl-Ethanolamin, N-Oleoyl-Ethanolamin, N-Linolenoyl-Ethanolamin, N-Cocoyl-Ethanolamin und N-Palmitinoyl-Ethanolamin umfaßt, wobei diese zuvor genannten speziellen Ethanolamine sowohl als Einzelsubstanz als auch als Gemisch von mehreren Ethanolaminen Anwendung finden. Ebenso kann die erfindungsgemäße Zusammensetzung als N-Acyl-Alkanolamin ein N-Acyl-2-Hydroxy-Propylamin umfassen, wobei dieses N-Acyl-2-Hydroxy-Propylamin insbesondere als Acylrest Fettsäuren aus Kokosfett und/oder Palmöl enthält. Die zuvor aufgeführten N-Acyl-Alkanolamine bewirken desweiteren, daß die Hautfeuchtigkeit erhöht und zusätzlich auf einen akzeptablen Wert stabilisiert werden.

Um bei der erfindungsgemäßen Zusammensetzung zu erreichen, daß diese eine besonders hohe Wirksamkeit in bezug auf den Auf- und Ausbau der physikalisch-chemischen Barriere der Haut besitzt, sieht eine weitere, besonders vorteilhafte Ausbildung der erfindungsgemäßen Zusammensetzung vor, daß diese ein Öl oder einen Ölbestandteil enthält, wobei dieses Öl bzw. der Ölbestandteil in der Zusammensetzung in einer Konzentration insbesondere zwischen 0,5 Gew.% und 10 Gew.%, bezogen auf die anwendungsfertige Zusammensetzung, vorhanden ist. Vorzugsweise wird hierbei als Öl bzw. als Ölbestandteil ein natürliches Öl und insbesondere ein pflanzliches Öl und vorzugsweise Avocadoöl, Kokosnußöl, Mandelöl und/oder Olivenöl ausgewählt, wobei es selbstverständlich möglich ist, daß die zuvor genannten Öle bzw. oder auch entsprechend hieraus isolierte Ölbestandteil jeweils allein und ebenso als Mischung in der erfindungsgemäßen Zusammensetzung enthalten sein können. Hierbei bewirken diese zuvor beschriebenen Inhaltsstoffe (Öl bzw. Ölbestandteile), daß neben dem Auf- und Ausbau der physikalisch-chemischen Hautbarriere die hiermit behandelte Haut vor schädigenden Umwelteinflüssen besonders geschützt wird.

Abhängig von der jeweiligen Art der Applikation, d.h. ob die erfindungsgemäße Zusammensetzung z.B. als Creme, Salbe, Gel, Lotion oder Zusatz zum Bad, verwendet wird, enthalten entsprechend formulierte Ausführungsformen der erfindungsgemäßen Zusammensetzung mindestens ein Konservierungsmittel, ein Antioxidanz, ein Verdickungsmittel und/oder einen Gelbildner, wobei die Konzentration dieser Konservierungsmittel und Antioxidantien, die nachfolgend noch zusammengefaßt als sonstige Zusätze bezeichnet sind, insbesondere zwischen 0 Gew.% und 10 Gew.%, bezogen auf die anwendungsfertige Zusammensetzung, variiert.

Ist in bevorzugten Ausführungsformen der erfindungsgemäßen Zusammensetzung ein Verdickungsmittel oder ein Gelbildner vorhanden, so bietet es sich hierbei an, als Gelbildner bzw. als Verdickungsmittel ein natürliches und/oder synthetisches Kolloid und/oder ein natürliches und/oder synthetisches Hydrokolloid auszuwählen, wobei es sehr wohl möglich ist, daß die erfindungsgemäße Zusammensetzung ein Gemisch aus natürlichem und synthetischem Verdickungsmittel bzw. aus natürlichem und synthetischem Gelbildner enthält. Die Konzentration dieser Kolloide bzw. Hydrokolloide variiert üblicherweise zwischen 0,1 Gew.% und 5 Gew.%, jeweils bezogen auf die anwendungsfertige Zusammensetzung.

Als Beispiel geeigneter Gelbildner bzw. Verdickungsmittel sind insbesondere die an sich bekannten Stärkeether, Stärkeester, Celluloseether oder Celluloseester oder aber auch die Derivate der Acrylsäure und/oder Derivate der Acrylsäuresalze, insbesondere oligomere und polymere Acrylsäure bzw. Acrylsäuresalze oder Derivate davon zu nennen.

Eine weitere Ausgestaltung der erfindungsgemäßen Zusammensetzung, die insbesondere dazu geeignet ist, die Interzellular-Lipide bei der Haut von Säuglingen bzw. Kleinkindern zu stärken, aus- und aufzubauen sowie den Säuremantel und das Hautoberflächenfett zu vergrößern und damit einen erhöhten Schutz der Haut zu bewirken, sieht vor, daß die Zusammensetzung mindestens eine Verbindung enthält, die aus der Gruppe, bestehend aus Sheabutter, Ceramide, insbesondere Ceramide-3, Cupuacu-Butter, Squalan und/oder Triglyceride, insbesondere C 18:1 Fettsäuretriglycerid, ausgewählt ist. Hierbei sind diese zuvor genannten Verbindungen in Kombination mit der lamellaren Struktur, die in der erfindungsgemäßen Zusammensetzung vorhanden ist, besonders geeignet, innerhalb der Interzellular-Kanälen eingelagert zu werden und dort die natürlich vorhandene Lipidphase zu verstärken.

Wie bereits vorstehend dargelegt ist, richten sich die Inhaltsstoffe der erfindungsgemäßen Zusammensetzung einerseits nach der jeweils erwünschten Anwendung und andererseits danach, welche zusätzlichen Zwecke mit der erfindungsgemäßen Zusammensetzung verfolgt werden sollen. Grundsätzlich enthält die erfindungsgemäßen Zusammensetzung mindestens einen anti-entzündlichen Wirkstoff, mindestens eine der zuvor beschriebenen Trägersubstanzen für den Wirkstoff sowie mindestens eine hydrophile Flüssigkeit, wobei die Trägersubstanz mit der hydrophilen Flüssigkeit lamellare, schichtartig ausgerichtete Doppelmembrane ausbildet, derart, daß innerhalb der Doppelmembrane der anti-entzündliche Wirkstoff homogen dispergiert ist.

Bevorzugte Ausführungsformen der erfindungsgemäßen Zusammensetzung weisen als Trägersubstanz jeweils zwischen 0,5 Gew.% und 7 Gew.% eines hydrierten Phosphatidylcholins, zwischen 0,01 Gew.% und 5 Gew.% des anti-entzündlichen Wirkstoffes und zwischen 5 Gew.% und 96 Gew.% Wasser als hydrophile Flüssigkeit auf, wobei dann diese bevorzugten Ausführungsformen desweiteren zwischen 0,5 Gew.% und 10 Gew.% Cupuacu Butter, zwischen 0,5 Gew.% und 15 Gew.% Sheabutter, zwischen 0,01 Gew.% und 3 Gew.% Ceramide-3, zwischen 0,1 Gew.% und 5 Gew.% des Kolloids oder Hydrokolloids, zwischen 2 Gew.% und 42 Gew.% des zuvor beschriebenen Öls und/oder des zuvor beschriebenen Ölanteils, sowie zwischen 0 Gew.% und 10 Gew.% sonstiger Zusätze enthalten.

Bezüglich des pH-Wertes, die die erfindungsgemäße Zusammensetzung aufweist, ist insbesondere festzuhalten, daß hier ein pH-Wert ausgewählt wird, der vorzugsweise zwischen 4,0 und 7,6 und insbesondere zwischen 4,8 und 7,2, variiert.

Wie bereits vorstehend mehrfach herausgestellt wurde, ist ein wesentliches Kriterium der erfindungsgemäßen Zusammensetzung, daß diese lamellare Doppelmembrane enthält und das desweiteren innerhalb der lamellaren Doppelmembrane der anti-entzündliche Wirkstoff homogen dispergiert ist. Insbesondere dann, wenn die erfindungsgemäße Zusammensetzung zwischen 10 Gew.% und 95 Gew.%, vorzugsweise zwischen 30 Gew.% und 95 Gew.% derartiger lamellarer Doppelmembranstrukturen enthält, wobei sich die zuvor angegebenen Konzentrationen auf das Gewicht der in der erfindungsgemäßen Zusammensetzung enthaltenen Trägersubstanz bezieht, weist eine derartige Ausgestaltung eine besonders hohe Aus- und Aufbauvermögen für die physikalisch-chemischen Hautbarriere auf, so daß diese Ausführungsformen besonders bevorzugt bei Säuglingen und Kleinkindern angewendet werden.

Diese bevorzugte Anwendung der erfindungsgemäßen Zusammensetzung bei Säuglingen, Kleinkindern und Kindern ist insbesondere dann auch gegeben, wenn die erfindungsgemäße Zusammensetzung eine solche lamellare Doppelmembranstruktur besitzt, bei der jede einzelne Doppelmembrane eine Dicke zwischen 4 nm und 20 nm, insbesondere zwischen 4 nm und 8 nm, aufweist.

Wie bereits vorstehend wiederholt dargelegt ist, kann die erfindungsgemäße Zusammensetzung in jeder geeigneten Form appliziert werden, sei es beispielsweise als Creme, Salbe, Gel, Lotion oder als Zusatz zu einem Bad.

Besonders geeignet ist es jedoch, wenn die erfindungsgemäße Zusammensetzung als cremeartige oder gelartige Zusammensetzung vorliegt und eine Viskosität bei 20 °C zwischen 2.000 mPas und 40.000 mPas, vorzugsweise zwischen 12.000 mPas und 25.000 mPas, besitzt.

Vorteilhafte Weiterbildungen der erfindungsgemäßen Zusammensetzung sind in den Unteransprüchen angegeben.

Die erfindungsgemäße Zusammensetzung wird nachfolgend anhand von fünf Ausführungsbeispielen in Verbindung mit der Zeichnung erläutert. Es zeigen:
- Figur 1: schematisch eine einzige Doppelmembranstruktur und
- Figur 2: schematisch eine Struktur von drei, sandwichartig übereinander angeordneten Doppelmembranen.

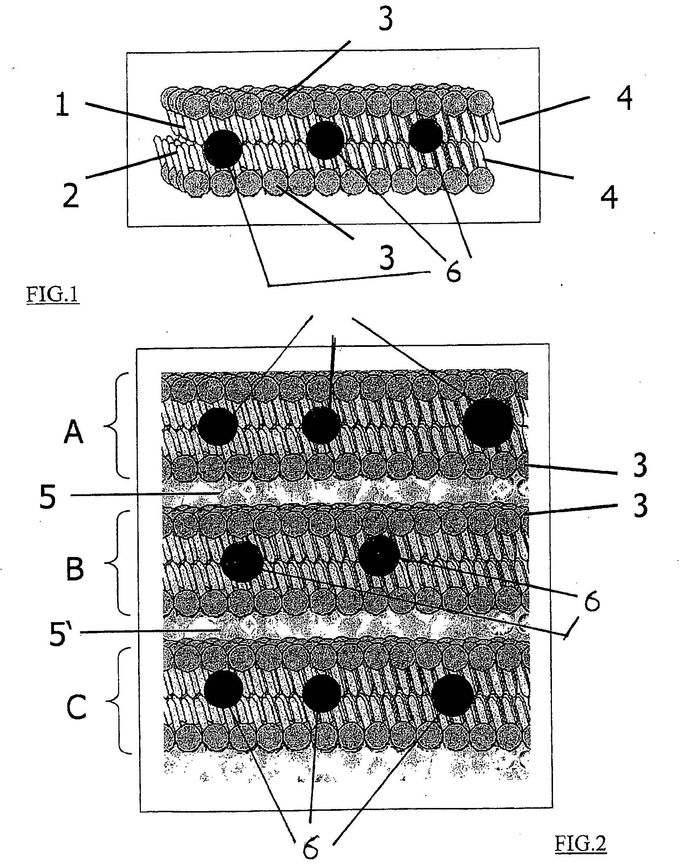

In den Figuren 1 und 2 sind jeweils lamellare Doppelmembranen schematisch abgebildet, die die Trägersubstanz in Verbindung mit Wasser ausbildet, wobei innerhalb der Trägersubstanz dann der anti-entzündliche Wirkstoff bzw. das anti-entzündliche Wirkstoffgemisch homogen dispergiert ist.

Wie den beiden Figuren 1 und 2 zu entnehmen ist, besitzt jede lamellare Doppelmembranstruktur einen Schichtaufbau, derart, daß eine erste Trägersubstanz-Schicht 1 parallel und planar zu einer zweiten Trägersubstanz-Schicht 2 ausgerichtet ist, wobei die hydrophilen Reste 3 einer jeden Trägersubstanz-Schicht 1 bzw. 2 jeweils nach außen und somit zu der die lamellare Doppelmembranstruktur umgebende hydrophile Flüssigkeitsschicht, insbesondere Wasserschicht bzw. Wasserschichten, ausgerichtet sind. Dementsprechend sind die lipophilen Reste 4 einer jeden lamellaren Doppelmembranstruktur nach innen hin positioniert. Zwischen den schichtartig angeordneten hydrophilen Resten 3 im Bereich der lipophilen Reste 4 ist der vorzugsweise mono- oder oligodispers ausgebildete anti-entzündliche Wirkstoff homogen verteilt, was schematisch durch das Bezugszeichen 6 sowohl in Figur 1 als auch in Figur 2 abgebildet ist.

Während die zuvor beschriebene Figur 1 eine einzelne Doppelmembranstruktur abbildet, sind in der Figur 2 schematisch drei Doppelmembranstrukturen, die vorstehend auch zusammengefaßt als Doppelmembrane bezeichnet sind, abgebildet. Hierbei besteht die in Figur 2 schematisch gezeigte Struktur aus drei, sandwichartig übereinander angeordnete Doppelmembranen A, B und C, wobei jede Doppelmembrane A, B und C einen solchen Aufbau aufweist, wie dieser vorstehend für die in Figur 1 gezeigte Doppelmembrane beschrieben wurde. Zwischen jeder Doppelmembrane A, B und C ist jeweils mindestens eine Schicht der hydrophilen Flüssigkeit und insbesondere mindestens eine Wasserschicht 5 bzw. 5' vorgesehen, die die lipophilen Reste 3 einer jeder Doppelmembrane auf Abstand hält, wie dies beispielsweise in Figur 2 für die Doppelmembrane A und die Doppelmembrane B gezeigt ist.

### Beispiel A

### Babycreme zur Anwendung für die Gesichtshaut

**Inhaltsstoffe der Phase 1**

| | |
|---|---|
| 2,000 Gew.% | Hydrogenated Lecithin |
| 1,250 Gew.% | Avocadoöl |
| 1,250 Gew.% | Sheabutter |
| 0,250 Gew.% | Squalane |
| 0,200 Gew.% | Caprylyl Glykol |
| 0,025 Gew.% | Ursolsäure Na-Salz |
| 0,050 Gew.% | Soy Sterol |
| 0,050 Gew.% | 18 beta Glycyrrhetinic Acid |
| 0,100 Gew.% | Ceramide 3 |

**Inhaltsstoff der Phase 2**

| | |
|---|---|
| 20,900 Gew.% | Osmosewasser |

**Inhaltsstoffe der Phase 3**

| | |
|---|---|
| 20,900 Gew.% | Caprylic / Capric Triglyceride |
| 10,000 Gew.% | Sheabutter |
| 3,500 Gew.% | Kokosnußöl, raffiniert |
| 3,500 Gew.% | C18:1 Fettsäure Triglycerid |
| 0,100 Gew.% | Carbomer |
| 0,100 Gew.% | Xanthan Gum |
| 0,100 Gew.% | Sodium Carbomer |

**Inhaltsstoffe der Phase 4**

| | |
|---|---|
| 23,875 Gew.% | Osmosewasser |
| 3,500 Gew.% | Pentylenen Glycol |
| 0,350 Gew.% | Hydroxyethylcellulose |
| 8,000 Gew.% | Glycerin |

Zur Herstellung des vorstehend genannten Ausführungsbeispieles A werden die Inhaltsstoffe der Phasen 1 und 2 auf 75 °C erhitzt und vermischt. Anschließend erfolgt ein Homogenisieren während zwei Minuten bei 800 bar unter Verwendung eines Hochdruckhomogenisators (5 Zyklen). Hiernach wird unter Rühren langsam auf 30 °C abgekühlt. Es schließt sich ein erneutes Homogenisieren während fünf Minuten bei 100 bar (1 Zyklus) an.

Nachdem die Hydroxyethylcellulose aus Phase 4 bei 50 °C vollständig quellengelassen worden ist und die Phase 3 auf 50 °C erwärmt wurde, um so die Sheabutter aufzuschmelzen und alle Gelbildner zu dispergieren, werden die vermischten Inhaltsstoffe der Phase 4 zu den vermischten Inhaltsstoffen der Phase 3 gegeben und hiernach zwei Minuten homogenisiert.

Unter Rühren werden die vereinten Phasen 3 und 4 auf 30 °C abgekühlt und mit den vereinten Phasen 1 und 2 vermischt, woran sich dann ein zehnminütiges Homogenisieren mit gleichzeitigem Abkühlen auf Raumtemperatur anschließt.

### Beispiel B

### Babycreme zur Anwendung bei Windeldermatitis

**Inhaltsstoffe der Phase 1**

| | |
|---|---|
| 2,000 Gew.% | Hydrogenated Lecithin |
| 0,500 Gew.% | Mandelöl |
| 2,000 Gew.% | Sheabutter |
| 0,750 Gew.% | Squalane |
| 0,200 Gew.% | Caprylyl Glycol |
| 0,500 Gew.% | Gamma Oryzanol |
| 0,500 Gew.% | Soy Sterol |
| 0,100 Gew.% | Ceramide 1 |

**Inhaltsstoffe der Phase 2**

| | |
|---|---|
| 20,900 Gew.% | Osmosewasser |
| 1,000 Gew.% | Panthenol |

**Inhaltsstoffe der Phase 3**

| | |
|---|---|
| 20,000 Gew.% | Caprylic / Capric Triglyceride |
| 15,000 Gew.% | Sheabutter |
| 1,000 Gew.% | ZnO |
| 0,150 Gew.% | Carbomer |
| 0,100 Gew.% | Xanthan Gum |
| 0,120 Gew.% | Sodium Carbomer |

**Inhaltsstoffe der Phase 4**

| | |
|---|---|
| 23,380 Gew.% | Osmosewasser |
| 3,500 Gew.% | Pentylenen Glycol |
| 0,300 Gew.% | Hydroxyethylcellulose |
| 8,000 Gew.% | Glycerin |

Zur Herstellung des vorstehend genannten Ausführungsbeispieles B werden die Inhaltsstoffe der Phasen 1 und 2 auf 75 °C erhitzt und vermischt. Anschließend erfolgt ein Homogenisieren während zwei Minuten bei 700 bar unter Verwendung eines Hochdruckhomogenisators (3 Zyklen). Hiernach wird unter Rühren langsam auf 30 °C abgekühlt. Es schließt sich ein erneutes Homogenisieren während fünf Minuten bei 100 bar (1 Zyklus) an.

Nachdem die Hydroxyethylcellulose aus Phase 4 bei 50 °C vollständig quellengelassen worden ist und die Phase 3 auf 50 °C erwärmt wurde, um so die Sheabutter aufzuschmelzen und alle Gelbildner und das Zinkoxid zu dispergieren, werden die vermischten Inhaltsstoffe der Phase 4 zu den vermischten Inhaltsstoffen der Phase 3 gegeben und hiernach zwei Minuten homogenisiert.

Unter Rühren werden die vereinten Phasen 3 und 4 auf 30 °C abgekühlt und mit den vereinten Phasen 1 und 2 vermischt, woran sich dann ein achtminütiges Homogenisieren mit gleichzeitigem Abkühlen auf Raumtemperatur anschließt.

### Beispiel C

### Babylotion für die Ganzkörperpflege

**Inhaltsstoffe der Phase 1**

| | |
|---|---|
| 0,900 Gew.% | Hydrogenated Lecithin |
| 0,500 Gew.% | Avocadoöl |
| 1,300 Gew.% | Olivenöl |
| 0,100 Gew.% | Squalane |
| 0,100 Gew.% | Caprylyl Glycol |
| 0,020 Gew.% | Ferula Säure |
| 0,030 Gew.% | Soy Sterol |
| 0,100 Gew.% | Ceramide 3 |

**Inhaltsstoffe der Phase 2**

| | |
|---|---|
| 20,900 Gew.% | Osmosewasser |
| 0,400 Gew.% | Urea |

**Inhaltsstoffe der Phase 3**

| | |
|---|---|
| 10,000 Gew.% | Caprylic / Capric Triglyceride |
| 3,500 Gew.% | Kokosnußöl, raffiniert |
| 0,050 Gew.% | Carbomer |
| 0,100 Gew.% | Xanthan Gum |
| 0,050 Gew.% | Sodium Carbomer |

**Inhaltsstoffe der Phase 4**

| | |
|---|---|
| 51,650 Gew.% | Osmosewasser |
| 4,000 Gew.% | Pentylenen Glycol |
| 0,300 Gew.% | Hydroxyethylcellulose |
| 6,000 Gew.% | Glycerin |

Zur Herstellung des vorstehend genannten Ausführungsbeispieles C werden die Inhaltsstoffe der Phasen 1 und 2 auf 75 °C erhitzt und vermischt. Anschließend erfolgt ein Homogenisieren während zwei Minuten bei 800 bar unter Verwendung eines Hochdruckhomogenisators (5 Zyklen). Hiernach wird unter Rühren langsam auf 30 °C abgekühlt. Es schließt sich ein erneutes Homogenisieren während fünf Minuten bei 100 bar (1 Zyklus) an.

Nachdem die Hydroxyethylcellulose aus Phase 4 bei 50 °C vollständig quellengelassen worden ist und die Phase 3 auf 40 °C erwärmt wurde, um so das Kokosöl aufzuschmelzen und alle Gelbildner zu dispergieren, werden die vermischten Inhaltsstoffe der Phase 4 zu den vermischten Inhaltsstoffen der Phase 3 gegeben und hiernach drei Minuten homogenisiert.

Unter Rühren werden die vereinten Phasen 3 und 4 auf 30 °C abgekühlt und mit den vereinten Phasen 1 und 2 vermischt, woran sich dann ein fünfminütiges Homogenisieren mit gleichzeitigem Abkühlen auf Raumtemperatur anschließt.

### Beispiel D

### Formulierung zur Anwendung als schützendes Babybad

**Inhaltsstoffe der Phase 1**

| | |
|---|---|
| 1,800 Gew.% | Hydrogenated Lecithin |
| 1,250 Gew.% | Lecithin |
| 10,000 Gew.% | Sheabutter |
| 5,400 Gew.% | Squalane |
| 23,000 Gew.% | Caprylic / Capric Triglyceride |
| 0,500 Gew.% | Ursolsäure Na-Salz |
| 0,900 Gew.% | Soy Sterol |
| 0,100 Gew.% | Ceramide 3 |

**Inhaltsstoffe der Phase 2**

| | |
|---|---|
| 42,050 Gew.% | Osmosewasser |
| 1,000 Gew.% | Serine |
| 0,800 Gew.% | Alanin |
| 6,000 Gew.% | Glycerin |
| 5,000 Gew.% | Pentylene Glycol |
| 2,000 Gew.% | Sorbitol |

Zur Herstellung des vorstehend genannten Ausführungsbeispieles D werden die Inhaltsstoffe der Phasen 1 und 2 auf 75 °C erhitzt und vermischt. Anschließend erfolgt ein Homogenisieren während zwei Minuten bei 800 bar unter Verwendung eines Hochdruckhomogenisators (5 Zyklen). Hiernach wird unter Rühren 'langsam auf 30 °C abgekühlt. Es schließt sich ein erneutes Homogenisieren während fünf Minuten bei 300 bar (2 Zyklen) an, wonach unter Rühren von 30 °C auf Raumtemperatur abgekühlt wird.

### Beispiel E

### Babycreme zur Anwendung bei extrem trockener und zu Ekzemen neigender Haut

**Inhaltsstoffe der Phase 1**

| | |
|---|---|
| 2,400 Gew.% | Hydrogenated Lecithin |
| 1,500 Gew.% | Cupuacu Butter |
| 1,000 Gew.% | Sheabutter |
| 1,200 Gew.% | Squalane |
| 0,220 Gew.% | Caprylyl Glycol |
| 0,025 Gew.% | Ursolsäure Na-Salz |
| 0,050 Gew.% | Soy Sterol |
| 0,300 Gew.% | Gamma Oryzanol |
| 0,100 Gew.% | Ceramide 3 |

**Inhaltsstoffe der Phase 2**

| | |
|---|---|
| 23,200 Gew.% | Osmosewasser |
| 1,000 Gew.% | Ectoin |

**Inhaltsstoffe der Phase 3**

| | |
|---|---|
| 18,600 Gew.% | Caprylic / Capric Triglyceride |
| 11,000 Gew.% | Sheabutter |
| 2,500 Gew.% | Kokosnußöl, raffiniert |
| 1,100 Gew.% | Cupuacu Butter |
| 0,080 Gew.% | Carbomer |
| 0,100 Gew.% | Xanthan Gum |
| 0,070 Gew.% | Sodium Carbomer |

**Inhaltsstoffe der Phase 4**

| | |
|---|---|
| 25,945 Gew.% | Osmosewasser |
| 3,200 Gew.% | Pentylenen Glycol |
| 0,310 Gew.% | Hydroxyethylcellulose |
| 6,000 Gew.% | Glycerin |
| 0,100 Gew.% | Pyrrolidone carboxylic Acid |

Zur Herstellung des vorstehend genannten Ausführungsbeispieles E werden die Inhaltsstoffe der Phasen 1 und 2 auf 75 °C erhitzt und vermischt. Anschließend erfolgt ein Homogenisieren während zwei Minuten bei 800 bar unter Verwendung eines Hochdruckhomogenisators (4 Zyklen). Hiernach wird unter Rühren langsam auf 30 °C abgekühlt. Es schließt sich ein erneutes Homogenisieren während fünf Minuten bei 100 bar (1 Zyklus) an.

Nachdem die Hydroxyethylcellulose aus Phase 4 bei 50 °C vollständig quellengelassen worden ist und die Phase 3 auf 50 °C erwärmt wurde, um so die Sheabutter und die Cupuacu Butter aufzuschmelzen und alle Gelbildner zu dispergieren, werden die vermischten Inhaltsstoffe der Phase 4 zu den vermischten Inhaltsstoffen der Phase 3 gegeben und hiernach drei Minuten homogenisiert.

Unter Rühren werden die vereinten Phasen 3 und 4 auf 30 °C abgekühlt und mit den vereinten Phasen 1 und 2 vermischt, woran sich dann ein zwölfminütiges Homogenisieren mit gleichzeitigem Abkühlen auf Raumtemperatur anschließt.

Die vorstehend angegebenen Inhaltsstoffe entsprechen der INCI-Terminologie. Soweit nicht ausdrücklich angegeben, sind alle Prozentangaben Gewichtsprozent-Angaben.

## Patentansprüche

1. Topisch zu applizierende Zusammensetzung zur Anwendung beim Säugling oder Kleinkind, die eine hydrophile Flüssigkeit, mindestens einen anti-entzündlichen Wirkstoff sowie mindestens eine Trägersubstanz für den Wirkstoff aufweist, wobei die Trägersubstanz mit der hydrophilen Flüssigkeit lamellare Strukturen ausbildet, **dadurch gekennzeichnet,**
a) **daß** die lamellaren Strukturen lamellare Doppelmembranstrukturen mit einem Schichtaufbau sind, bei denen eine erste Trägersubstanzschicht parallel und planar zu einer zweiten Trägersubstanzschicht ausgerichtet ist und die hydrophilen Reste einer jeden Trägersubstanzschicht nach außen und die lipophilen Reste einer jeden Trägersubstanzschicht nach innen hin positioniert sind,
b) **daß** zwischen den schichtartig angeordneten hydrophilen Resten im Bereich der lipophilen Reste der anti-entzündliche Wirkstoff homogen mono- oder oligodispers verteilt ist,
c) **daß** die Zusammensetzung als Trägersubstanz ein hydriertes Phospholipid mit einer Phasenübergangstemperatur über 30 °C und unter 70 °C aufweist, und
d) **daß** die Zusammensetzung als Wirkstoff einen anti-entzündlichen Wirkstoff aufweist, der aus der Gruppe ausgewählt ist, die Ursolsäure, Soja-Sterol, 18-beta-Glycyrrhetinsäure, Gamma-Oryzanol, Ferula-Säure umfaßt.

2. Zusammensetzung nach Anspruch 1, **dadurch gekennzeichnet, daß** der anti-entzündliche Wirkstoff ein in der hydrophilen Flüssigkeit schlecht löslicher Wirkstoff ist.

3. Zusammensetzung nach Anspruch 1 oder 2, **dadurch gekennzeichnet, daß** der anti-entzündliche Wirkstoff in der Trägersubstanz schlecht löslich ist.

4. Zusammensetzung nach einem der vorangehenden Ansprüche, **dadurch gekennzeichnet, daß** die Zusammensetzung den anti-entzündlichen Wirkstoff in einer Konzentration zwischen 0,01 Gew.% und 5 Gew.%, vorzugsweise zwischen 0,1 Gew.% und 2,5 Gew.%, bezogen auf die anwendungsfertige Zusammensetzung aufweist.

5. Zusammensetzung nach einem der vorangehenden Ansprüche, **dadurch gekennzeichnet, daß** die hydrophile Flüssigkeit Wasser ist.

6. Zusammensetzung nach einem der vorangehenden Ansprüche, **dadurch gekennzeichnet, daß** die Zusammensetzung als Trägersubstanz ein hydriertes Phosphatidylcholin enthält.

7. Zusammensetzung nach Anspruch 6, **dadurch gekennzeichnet, daß** das hydrierte Phosphatidylcholin wenigstens 60 Gew.% hydriertes Phosphatidylcholin aufweist.

8. Zusammensetzung nach einem der vorangehenden Ansprüche, **dadurch gekennzeichnet, daß** die Zusammensetzung die Trägersubstanz in einer Konzentration zwischen 0,5 Gew.% und 30 Gew.%, vorzugsweise in einer Konzentration zwischen 0,7 Gew.% und 5 Gew.%, bezogen auf die anwendungsfertige Zusammensetzung, aufweist.

9. Zusammensetzung nach einem der vorangehenden Ansprüche, **dadurch gekennzeichnet, daß** die Zusammensetzung einen weiteren Wirkstoff enthält, der aus der Gruppe ausgewählt ist, die Antiallergika, Antibiotika, Antimykotika, Dermatika, Antiseptika, durchblutungsfördernde Wirkstoffe, Vitamine und Wundbehandlungsmittel jeweils allein oder in Mischung umfaßt.

10. Zusammensetzung nach Anspruch 9, **dadurch gekennzeichnet, daß** der weitere Wirkstoff ein lokal wirkendes Dermatikum ist.

11. Zusammensetzung nach einem der vorangehenden Ansprüche, **dadurch gekennzeichnet, daß** der weitere Wirkstoff in der Zusammensetzung in einer Konzentration zwischen 0,05 % und 20 %, vorzugsweise in eine Konzentration zwischen 0,1 % und 4 %, bezogen auf das Gewicht der anwendungsfertigen Zusammensetzung, vorhanden ist.

12. Zusammensetzung nach einem der vorangehenden Ansprüche, **dadurch gekennzeichnet, daß** die Zusammensetzung Wasser in einer Konzentration zwischen 5 % und 90 %, bezogen auf das Gewicht der anwendungsfertigen Zusammensetzung aufweist.

13. Zusammensetzung nach einem der vorangehenden Ansprüche, **dadurch gekennzeichnet, daß** die Zusammensetzung desweiteren mindestens einen Alkohol, insbesondere einen mehrwertigen Alkohol, aufweist.

14. Zusammensetzung nach Anspruch 13, **dadurch gekennzeichnet, daß** die Zusammensetzung als Alkohol Pentylenglykol, Caprylyl Glykol und/oder Glycerin aufweist.

15. Zusammensetzung einem der vorangehenden Ansprüche, **dadurch gekennzeichnet, daß** die Zusammensetzung ein N-Acyl-Ethanolamin in einer Konzentration zwischen 0,01 % und 10 %, vorzugsweise zwischen 0,1 % und 3 %, bezogen auf das Gewicht der anwendungsfertigen Zusammensetzung aufweist.

16. Zusammensetzung nach Anspruch 15, **dadurch gekennzeichnet, daß** der Acylrest des N-Acyl-Ethanolamins ein C₁-C₁₈-Acylrest ist.

17. Zusammensetzung nach Anspruch 15 oder 16, **dadurch gekennzeichnet, daß** das N-Acyl-Ethanolamin aus der Gruppe ausgewählt ist, die N-Acetyl-Ethanolamin, N-Oleoyl-Ethanolamin, N-Linolenoyl-Ethanolamin, N-Cocoyl-Ethanolamin und N-Palmitinoyl-Ethanolamin umfaßt.

18. Zusammensetzung nach einem der vorangehenden Ansprüche, **dadurch gekennzeichnet, daß** die Zusammensetzung ein Öl und/oder einen Ölbestandteil, insbesondere Avocadoöl, Kokosnußöl, Mandelöl und/oder Olivenöl, aufweist.

19. Zusammensetzung nach Anspruch 18, **dadurch gekennzeichnet, daß** das Öl bzw. der Ölbestandteil in der Zusammensetzung in einer Konzentration zwischen 0,5 Gew.% und 10 Gew.%, bezogen auf die anwendungsfertige Zusammensetzung, vorhanden ist.

20. Zusammensetzung nach einem der vorangehenden Ansprüche, **dadurch gekennzeichnet, daß** die Zusammensetzung desweiteren mindestens ein Konservierungsmittel, ein Antioxidanz, ein Verdickungsmittel und/oder einen Gelbildner aufweist.

21. Zusammensetzung nach Anspruch 20, **dadurch gekennzeichnet, daß** die Zusammensetzung als Verdickungsmittel bzw. als Gelbildner ein natürliches Kolloid oder ein natürliches Hydrokolloid und/oder ein synthetisches Kolloid oder ein synthetisches Hydrokolloid aufweist.

22. Zusammensetzung nach einem der vorangehenden Ansprüche, **dadurch gekennzeichnet, daß** die Zusammensetzung Sheabutter, Ceramide, insbesondere Ceramide-3, Cupuacu-Butter, Squalan und/oder Triglyceride, insbesondere C 18:1 Fettsäuretriglycerid, enthält.

23. Zusammensetzung nach einem der vorangehenden Ansprüche, **dadurch gekennzeichnet, daß** die Zusammensetzung jeweils zwischen
0,5 Gew.% und 7 Gew.% hydriertes Phosphatidylcholin,
0,5 Gew.% und 10 Gew.% Cupuacu Butter
0,5 Gew.% und 15 Gew.% Sheabutter
0,01 Gew.% und 3 Gew.% Ceramide 3
0,1 Gew.% und 5 Gew.% des Kolloids oder Hydrokolloids
2 Gew.% und 42 Gew.% des Öls oder des Ölanteils
0,01 Gew.% und 5 Gew.% des anti-entzündlichen Wirkstoffs
0 Gew.% und 10 Gew.% sonstiger Zusätze und
5 Gew.% und 96 Gew.% Wasser
aufweist.

24. Zusammensetzung nach einem der vorangehenden Ansprüche, **dadurch gekennzeichnet, daß** die Zusammensetzung als topisch applizierbares Zusammensetzung formuliert ist und eine Viskosität bei 20 °C zwischen 2.000 mPas und 40.000 mPas, vorzugsweise zwischen 12.000 mPas und 25.000 mPas, aufweist.

25. Zusammensetzung nach einem der vorangehenden Ansprüche, **dadurch gekennzeichnet, daß** die Zusammensetzung einen pH-Wert zwischen 4,0 und 7,6 besitzt.

26. Zusammensetzung nach einem der vorangehenden Ansprüche, **dadurch gekennzeichnet, daß** die Zusammensetzung die lamellare Doppelmembranstruktur in einer Konzentration zwischen 10 % und 95 %, vorzugsweise zwischen 30 % und 95 %, bezogen auf das Gewicht der in der Zusammensetzung enthaltenen Trägersubstanz aufweist.

27. Zusammensetzung nach einem der vorangehenden Ansprüche, **dadurch gekennzeichnet, daß** die Zusammensetzung eine solche lamellare Doppelmembranstruktur aufweist, bei der jede einzelne Doppelmembrane eine Dicke zwischen 4 nm und 20 nm, vorzugsweise zwischen 4 nm und 8 nm, besitzt.

## Claims

1. Composition for topical application for use in infants or toddlers which has a hydrophilic liquid, at least one anti-inflammatory active agent and at least one carrier substance for said agent, wherein the carrier substance forms lamellar structures with the hydrophilic liquid, **characterized in**
a) **that** the lamellar structures are lamellar double membrane structures having a layer structure in which a first carrier substance layer is aligned in parallel and planarly to a second carrier substance layer and the hydrophilic residues of each carrier substance layer are positioned outwardly and the lipophilic residues of each carrier substance layer are positioned inwardly,
b) **that** between the hydrophilic residues which are arranged in layers, the anti-inflammatory agent is homogeneously distributed in a monodispersed or oligodispersed manner in the range of the lipophilic residues,
c) **that** the composition has a hydrogenated phospholipid having a phase transition temperature above 30°C and below 70°C as carrier substance, and
d) **that** the composition has an anti-inflammatory active agent as active agent selected from the group comprising ursolic acid, soy sterol, 18-beta-glycyrrhetinic acid, gamma-oryzanol, ferulic acid.

2. Composition according to claim 1, **characterized in that** the anti-inflammatory active agent is an active agent which is poorly soluble in the hydrophilic liquid.

3. Composition according to claim 1 or 2, **characterized in that** the anti-inflammatory active agent is poorly soluble in the carrier substance.

4. Composition according to any one of the preceding claims, **characterized in that** the composition contains the anti-inflammatory active agent in a concentration of between 0.01 wt.% and 5 wt.%, preferably between 0.1 wt.% and 2.5 wt.%, based on the ready-to-use composition.

5. Composition according to any one of the preceding claims, **characterized in that** the hydrophilic liquid is water.

6. Composition according to any one of the preceding claims, **characterized in that** the composition contains a hydrogenated phosphatidyl choline as carrier substance.

7. Composition according to claim 6, **characterized in that** the hydrogenated phosphatidyl choline contains at least 60 wt.% of hydrogenated phosphatidyl choline.

8. Composition according to any one of the preceding claims, **characterized in that** the composition contains the carrier substance in a concentration of between 0.5 wt.% and 30 wt.%, preferably in a concentration of between 0.7 wt.% and 5 wt.%, based on the ready-to-use composition.

9. Composition according to any one of the preceding claims, **characterized in that** the composition contains a further active agent selected from the group comprising antiallergics, antibiotics, antimycotics, dermatological agents, antiseptics, blood circulation-promoting active agents, vitamins and wound treating agents alone or in a mixture.

10. Composition according to claim 9, **characterized in that** the further active agent is a locally effective dermatological agent.

11. Composition according to any one of the preceding claims, **characterized in that** the further active agent is present in the composition in a concentration of between 0.05 % and 20 %, preferably in a concentration of between 0.1 % and 4 %, based on the weight of the ready-to-use composition.

12. Composition according to any one of the preceding claims, **characterized in that** the composition contains water in a concentration of between 5 % and 90 %, based on the weight of the ready-to-use composition.

13. Composition according to any one of the preceding claims, **characterized in that** the composition further contains at least one alcohol, in particular a polyvalent alcohol.

14. Composition according to claim 13, **characterized in that** the composition has pentylene glycol, caprylyl glycol and/or glycerine as alcohol.

15. Composition according to any one of the preceding claims, **characterized in that** the composition contains an N-acyl-ethanolamine in a concentration of between 0.01 % and 10 %, preferably between 0.1 % and 3 %, based on the weight of the ready-to-use composition.

16. Composition according to claim 15, **characterized in that** the acyl group of the N-acyl-ethanolamine is a C₁-C₁₈ acyl group.

17. Composition according to claim 15 or 16, **characterized in that** the N-acyl-ethanolamine is selected from the group comprising N-acetyl-ethanolamine, N-oleoyl-ethanolamine, N-linolenoyl-ethanolamine, N-cocoyl-ethanolamine and N-palmitinoyl-ethanolamine.

18. Composition according to any one of the preceding claims, **characterized in that** the composition contains an oil and/or oil component, in particular avocado oil, coconut oil, almond oil and/or olive oil.

19. Composition according to claim 18, **characterized in that** the oil and the oil component, respectively, is present in the composition in a concentration of between 0.5 wt.% and 10 wt.%, based on the ready-to-use composition.

20. Composition according to any one of the preceding claims, **characterized in that** the composition further has at least one preservative, an antioxidant, a thickener and/or a gel-forming agent.

21. Composition according to claim 20, **characterized in that** the composition contains a natural colloid or a natural hydrocolloid and/or a synthetic colloid or a synthetic hydrocolloid as thickener or gel-forming agent.

22. Composition according to any one of the preceding claims, **characterized in that** the composition contains shea butter, ceramides, in particular ceramide-3, cupuaçu butter, squalane and/or triglycerides, in particular C 18:1 fatty acid triglyceride.

23. Composition according to any one of the preceding claims, **characterized in that** the composition each contains between
0.5 wt.% and 7 wt.% of hydrogenated phosphatidyl choline,
0.5 wt.% and 10 wt.% of cupuaçu butter,
0.5 wt.% and 15 wt.% of shea butter,
0.01 wt.% and 3 wt.% of ceramide-3,
0.1 wt.% and 5 wt.% of the colloid or hydrocolloid,
2 wt.% and 42 wt.% of the oil or the oil portion,
0.01 wt.% and 5 wt.% of the anti-inflammatory active agent,
0 wt.% and 10 wt.% of other additives and
5 wt.% and 96 wt.% of water.

24. Composition according to any one of the preceding claims, **characterized in that** the composition is formulated as a composition for topical application and that it has a viscosity at 20 °C of between 2,000 mPas and 40,000 mPas, preferably between 12,000 mPas and 25,000 mPas.

25. Composition according to any one of the preceding claims, **characterized in that** the composition has a pH of between 4.0 and 7.6.

26. Composition according to any one of the preceding claims, **characterized in that** the composition contains the lamellar double membrane structure in a concentration of between 10 % and 95 %, preferably between 30 % and 95 %, based on the weight of the carrier substance contained in the composition.

27. Composition according to any one of the preceding claims, **characterized in that** the composition has such a lamellar double membrane structure in which every single double membrane has a thickness of between 4 nm and 20 nm, preferably between 4 nm and 8 nm.

## Revendications

1. Composition à application topique destinée à être utilisée chez le nourrisson ou l'enfant en bas âge, qui comprend un liquide hydrophile, au moins une substance active anti-inflammatoire et au moins une substance support pour la substance active, dans laquelle la substance support forme des structures lamellaires avec le liquide hydrophile, **caractérisée**
a) **en ce que** les structures lamellaires sont des structures lamellaires à double membrane ayant une architecture en couches, dans lesquelles une première couche de substance support est orientée parallèlement et de manière plane par rapport à une deuxième couche de substance support, et les résidus hydrophiles de chaque couche de substance support sont positionnés vers l'extérieur et les résidus lipophiles de chaque couche de substance support sont positionnés vers l'intérieur,
b) **en ce que** la substance active anti-inflammatoire présente une distribution homogène monodispersée ou oligodispersée entre les résidus hydrophiles disposés en couche dans la zone des résidus lipophiles,
c) **en ce que** la composition comprend comme substance support un phospholipide hydrogéné ayant une température de transition de phase supérieure à 30 °C et inférieure à 70 °C, et
d) **en ce que** la composition présente comme substance active une substance active anti-inflammatoire, qui est choisie dans le groupe comprenant l'acide ursolique, les stérols de soja, l'acide 18-bêta-glycyrrhétinique, le gamma-oryzanol, l'acide férulique.

2. Composition selon la revendication 1, **caractérisée en ce que** la substance active anti-inflammatoire est une substance active difficilement soluble dans le liquide hydrophile.

3. Composition selon la revendication 1 ou 2, **caractérisée en ce que** la substance active anti-inflammatoire est difficilement soluble dans la substance support.

4. Composition selon l'une des revendications précédentes, **caractérisée en ce que** la composition comprend la substance active anti-inflammatoire en une concentration comprise entre 0,01 % en poids et 5 % en poids, de préférence entre 0,1 % en poids et 2,5 % en poids, par rapport à la composition prête à l'emploi.

5. Composition selon l'une des revendications précédentes, **caractérisée en ce que** le liquide hydrophile est l'eau.

6. Composition selon l'une des revendications précédentes, **caractérisée en ce que** la composition comprend comme substance support une phosphatidylcholine hydrogénée.

7. Composition selon la revendication 6, **caractérisée en ce que** la phosphatidylcholine hydrogénée comprend au moins 60 % en poids de phosphatidylcholine hydrogénée.

8. Composition selon l'une des revendications précédentes, **caractérisée en ce que** la composition comprend la substance support en une concentration comprise entre 0,5 % en poids et 30 % en poids, de préférence en une concentration comprise entre 0,7 % en poids et 5 % en poids, par rapport à la composition prête à l'emploi.

9. Composition selon l'une des revendications précédentes, **caractérisée en ce que** la composition comprend une autre substance active, qui est choisie dans le groupe comprenant des anti-allergiques, des antibiotiques, des anti-mycosiques, des agents dermatologiques, des antiseptiques, des substances actives améliorant la circulation sanguine, des vitamines et des agents cicatrisants, respectivement seuls ou en mélange.

10. Composition selon la revendication 9, **caractérisée en ce que** l'autre substance active est un agent dermatologique à action locale.

11. Composition selon l'une des revendications précédentes, **caractérisée en ce que** l'autre substance active est présente dans la composition en une concentration comprise entre 0,05 % et 20 %, de préférence en une concentration comprise entre 0,1 % et 4 %, par rapport au poids de la composition prête à l'emploi.

12. Composition selon l'une des revendications précédentes, **caractérisée en ce que** la composition comprend de l'eau en une concentration comprise entre 5 % et 90 %, par rapport au poids de la composition prête à l'emploi.

13. Composition selon l'une des revendications précédentes, **caractérisée en ce que** la composition comprend en outre au moins un alcool, en particulier un alcool polyvalent.

14. Composition selon la revendication 13, **caractérisée en ce que** la composition comprend comme alcool du pentylène glycol, du caprylyl glycol, et/ou de la glycérine.

15. Composition selon l'une des revendications précédentes, **caractérisée en ce que** la composition comprend une N-acyl-éthanolamine en une concentration comprise entre 0,01 % et 10 %, de préférence entre 0,1 % et 3 %, par rapport au poids de la composition prête à l'emploi.

16. Composition selon la revendication 15, **caractérisée en ce que** le résidu acyle de la N-acyl-éthanolamine est un résidu acyle en C₁-C₁₈.

17. Composition selon la revendication 15 ou 16, **caractérisée en ce que** la N-acyl-éthanolamine est choisie dans le groupe qui comprend la N-acétyl-éthanolamine, la N-oléoyl-éthanol aminé, la N-linolénoyl-éthanolamine, la N-cocoyl-éthanolamine et la N-palmitinoyl-éthanolamine.

18. Composition selon l'une des revendications précédentes, **caractérisée en ce que** la composition comprend une huile et/ou un composant huileux, en particulier de l'huile d'avocat, de l'huile de noix de coco, de l'huile d'amande douce et/ou de l'huile d'olive.

19. Composition selon la revendication 18, **caractérisée en ce que** l'huile ou le composant huileux est présent dans la composition en une concentration comprise entre 0,5 % en poids et 10 % en poids, par rapport à la composition prête à l'emploi.

20. Composition selon l'une des revendications précédentes, **caractérisée en ce que** la composition comprend en outre au moins un conservateur, un antioxydant, un agent épaississant et/ou un gélifiant.

21. Composition selon la revendication 20, **caractérisée en ce que** la composition comprend comme agent épaississant ou comme gélifiant un colloïde naturel ou un hydrocolloïde naturel et/ou un colloïde synthétique ou un hydrocolloïde synthétique.

22. Composition selon l'une des revendications précédentes, **caractérisée en ce que** la composition comprend du beurre de karité, un céramide, en particulier un céramide-3, du beurre de cupuaçu, du squalane, et/ou des triglycérides, en particulier un triglycéride d'acide gras en C 18:1.

23. Composition selon l'une des revendications précédentes, **caractérisée en ce que** la composition comprend respectivement entre
0,5 % en poids et 7 % en poids de phosphatidylcholine hydrogénée,
0,5 % en poids et 10 % en poids de beurre de cupuaçu,
0,5 % en poids et 15 % en poids de beurre de karité,
0,01 % en poids et 3 % en poids de céramide-3
0,1 % en poids et 5 % en poids de colloïde ou d'hydrocolloïde
2 % en poids et 42 % en poids d'huile ou de fraction huileuse,
0,01 % en poids et 5 % en poids de la substance active anti-inflammatoire
0 % en poids et 10 % en poids d'autres additifs et
5 % en poids et 96 % en poids d'eau.

24. Composition selon l'une des revendications précédentes, **caractérisée en ce que** la composition est formulée en tant que composition applicable topiquement et présente une viscosité à 20 °C comprise entre 2000 mPas et 40 000 mPas, de préférence entre 12 000 mPas et 25 000 mPas.

25. Composition selon l'une des revendications précédentes, **caractérisée en ce que** la composition possède un pH compris entre 4,0 et 7,6.

26. Composition selon l'une des revendications précédentes, **caractérisée en ce que** la composition comprend la structure lamellaire à double membrane en une concentration comprise entre 10 % et 95 %, de préférence entre 30 % et 95 %, par rapport au poids de la substance support contenue dans la composition.

27. Composition selon l'une des revendications précédentes, **caractérisée en ce que** la composition comprend une structure lamellaire à double membrane du type dans lequel chaque double membrane individuelle possède une épaisseur comprise entre 4 nm et 20 nm, de préférence entre 4 nm et 8 nm.
